# EUROPEAN PATENT SPECIFICATION

(11) **EP 0 932 594 B1**
(45) Date of publication and mention of the grant of the patent: **24.07.2002**
(21) Application number: 97911613.4
(22) Date of filing: 02.10.1997
(51) Int. Cl.: C07C 51/00

(54) **A PROCESS FOR THE RECOVERY OF LACTIC ACID**
VERFAHREN ZUR GEWINNUNG VON MILCHSAÜREESTERN UND AMIDEN AUS WÄSSRIGEN LÖSUNGEN VON MILCHSÄURE UND/ODER DEREN SALZEN
PROCEDE DE RECUPERATION DE L'ACIDE LACTIQUE

(30) Priority: 09.10.1996 IL 11938896
(43) Date of publication of application: 04.08.1999
(73) Proprietor: Cargill Incorporated, Wayzata, Minnesota 55391 (US)
(72) Inventor: EYAL, Aharon, Meir, 93380 Jerusalem (IL); GRUBER, Patrick, R., Blaine, MN 55449 (US); MCWILLIAMS, Paul, Racine, WI 53402 (US); WITZKE, David, R., Oskaloosa, IA 52577 (US)
(74) Representative: Ebner von Eschenbach, Jennifer
(86) International application number: US9717775
(87) International publication number: WO9815519

(56) References cited:
- EP-A- 0 517 571
- WO-A-97/30964
- US-A- 5 132 456
- US-A- 5 252 473
- DATABASE WPI Section Ch, Week 9549 Derwent Publications Ltd., London, GB; Class B05, AN 95380018 XP002059567 & JP 07 258 154 A (NITTO CHEM IND CO LTD) , 9 October 1995

## Description

The present invention relates to a process for the recovery of lactic acid. More particularly, the present invention relates to a process for the recovery of lactic acid from an aqueous solution containing lactic acid, lactate salts, or mixtures thereof.

Lactic acid has long been used as a food additive and in various chemical and pharmaceutical applications. More recently, lactic acid has been used in the making of biodegradable polylactic acid polymers as a replacement for present plastic materials, as well as for various new uses where biodegradability is needed or desired. Accordingly, there is an ever-increasing demand for lactic acid. The present invention aims at meeting this demand by providing an efficient and environmentally friendly process for producing lactic acid which avoids the consumption of bases and acids and substantially reduces, if not eliminates, the formation of waste or byproduct salts.

The production of lactic acid is commonly carried out by fermentation of a strain of the bacterial genus *Lactobacillus,* and more particularly, for example, by the species *Lactobacillus delbrueckii* or *Lactobacillus acidophilus.* In general, the production of lactic acid by fermentation in a fermentation broth is well known in the art. The fermentation substrate consists of carbohydrates together with suitable mineral and proteinaceous nutrients. Because the lactic acid-producing microorganisms are inhibited in a strongly acidic environment, the pH of the fermentation broth is usually kept above 4.5, preferably within the range of about 5.0 to 7.0, more preferably within the range of about 5.5 to 6.5, and most preferably within the range of about 6.0 to 6.5., although fermentation in a pH range of about 3.8-4.5 has also been carried out. To maintain this pH level, suitable water-soluble basic substances or agents that are non-toxic to the acid-producing microorganism, such as alkali metal hydroxides, carbonates, or bicarbonates, or alkaline earth metal hydroxides or carbonates, are commonly added to the fermentation broth to neutralize the acid being produced. This results in the formation of a lactate solution rather than the desired lactic acid product. Such a lactate solution contains the lactate anion and the corresponding cation of the substance used to neutralize the fermentation broth.

Various methods have been proposed for the recovery of lactic acid from a fermentation broth. When the fermentation is carried out in the presence of calcium carbonate, it is possible to recover the lactic acid by acidification with sulfuric acid. This results in the precipitation of calcium sulfate, while free lactic acid remains in the mother liquor. If desired, the mother liquor may be concentrated to up to about 90 wt% lactic acid. Subsequently, lactic acid may be extracted from the mother liquor with a suitable organic extractant, to yield an extract which is back-extracted with water, or the acid may be adsorbed on a suitable adsorbent and later desorbed. The resulting aqueous lactic acid solution may then be concentrated. This method has the disadvantage that it irreversibly consumes calcium carbonate and sulfuric acid and leaves, as waste, large quantities of calcium sulfate which give rise to disposal problems.

U.S. Patent No. 5,132,456 (King, et al.), describes a process for recovering carboxylic acid from a carboxylic acid-containing aqueous feed stream having a pH dose to or above the pKₐ level of the acid. The recovery of that process involves what may be described as a cascade-type acid withdrawal operation, in which the basicity of the extractant is increased stepwise. In a first stage of the process, the feed stream is contacted with an adsorbent such as a strongly basic extractant or a solid anion exchanger. In a second stage, the acid-loaded adsorbent is contacted with an aqueous solution of ammonia or a low molecular weight trialkyl amine having a stronger affinity to the carboxylic acid that is being recovered than that of the adsorber used in the first stage. In this way, an aqueous solution of a water-soluble carboxylic acid ammonium salt is formed. This is then subjected to heat treatment, whereby the salt is decomposed to yield back the trialkyl amine or ammonia and free carboxylic acid. Applying this process to lactic acid involves the formation of salts of lactic acid with strong bases having a pKₐ value of about 9-11. Thus, the decomposition of these salts into free lactic acid is energy intensive. Examples 12-14 of said U.S. patent mention the use of Alamine 336 (tricaprylylamine) for the extraction of, among others, lactic acid from an aqueous solution, but no yields are mentioned. By the extraction of even small quantities of lactic acid from a fermentation broth, the pH of the broth rises rapidly to above 7 while the pKa of an extractant based on Alamine 336 is less than 6. As shown in Figures 3 and 4 of said patent, the uptake of carboxylic acids from aqueous solutions drops rapidly with an increase of the pH. It is therefore inherent in these examples that the lactic acid uptake, if any, is negligible. It is further noted that upon heat treatment and concentration of an ammonium lactate, crystalline lactic add does not precipitate; instead, the viscosity of the solutions increases steadily as a result of self-association of the acid. It is thus evident that the process of U.S. 5,132,456 is unsuitable for the recovery of lactic acid from a fermentation broth.

U.S. Patents Nos. 4,444,881 and 4,405,717 (Urbas) describe a process for the recovery of an organic acid from a diluted aqueous solution of its calcium salt by adding a water-soluble trialkyl amine carbonate to the solution, to form, on the one hand, a water soluble trialkyl ammonium salt of the acid, which salt remains in solution; and, on the other hand, calcium carbonate which precipitates. After removal of the calcium carbonate, the remaining mother liquor is heated for the separate recovery of the amine and the product acid. The water-soluble trialkyl amines employed in accordance with these patents are strongly basic. Accordingly, the decomposition of the trialkylammonium salts into free acids is energy-intensive.

U.S. Patent No. 4,282,323 (Yates) describes a process for obtaining lower carboxylic acids from a salt solution of carboxylic acid obtained from fermentation. The process appears to be applicable to a restricted number of lower aliphatic and aromatic monocarboxylic acids and is specifically described only in relation to acetic acid. In accordance with that process, the aqueous solution of a carboxylic acid salt is contacted with pressurized carbon dioxide in the presence of a liquid polar organic solvent having a boiling point of from -30C to 90C serving as extractant, to convert at least part of the salt to the corresponding free acid, which is then taken up by the organic phase, from where it is subsequently recovered. It is inherent in the use of a polar organic extractant that the bulk of the carboxylic acid remains in the neutral to basic aqueous phase, and indeed the recovery rates reported in Patent No. 4,282,323 are low, ranging between 4.8-18% of the acid initially present.

U.S. Patent No. 4,275,234 (Baniel, et al.) is directed to a method of recovering various acids in their free form from aqueous solutions. Thus, the process of Baniel is not applicable to a lactate solution of the type commonly obtained from a fermentation process conducted at a pH higher than the pKa of the acid or from other sources. The essence of the Baniel patent is the discovery that efficient back-extraction can be achieved by performing the back-extraction at a temperature higher than that of the primary extraction.

R Bar and J.1. Geiner, Biotechnology Progress, Vol. 3, p. 109 (1987) studied the feasibility of extracting lactic acid from aqueous solution by means of a long-chain trialkyl amine of low basicity, such as tridodecylamine, using various tridodecylamine solutions in n-dodecanol. It was found that extraction of lactic acid with a long-chain trialkyl amine such as tridodecylamine was effective only at a pH that is lower than the pKa of lactic acid, the latter of which is 3.86. At such a low pH, however, the lactic acid fermenting microorganism such as, for example, *Lactobacillus delbrueckii* or *Lactobacillus acidophilus* is severely inhibited.

Free lactic acid can also be purified by forming its esters through condensation, purifying those esters by distillation or by extraction, and then hydrolyzing them back to lactic acid. Such condensation is usually catalyzed by an acidic medium.

As indicated above, lactic acid fermentation is conducted at about neutral pH. Since the pKa of lactic acid is 3.86, at the pH of fermentation, mostly lactate salts exist. Substantially complete recovery and purification of lactic acid from the fermentation liquor through esterification may require acidulation by a strong acid that displaces lactic acid from its salt and frees it to react with an alkanol to form the ester. Nakanishi and Tsuda (JP 46/30176) consider production of 1-butyl lactate by extraction of an acidified crude fermentation broth with 1-butanol, followed by esterification of the extract phase. BASF (EP 159585) considers a similar process with isobutanol to form isobutyl lactate.

In WO 93/00440, assigned to DuPont Corporation, there is described a process which comprises the steps of: (1) simultaneously mixing a strong acid, an alcohol, and a concentrated fermentation broth which contains mainly basic salts of lactic acid, which react to form a crystal precipitate comprising basic salts of the strong acid and an impure lactate ester of the alcohol; (2) removing water from the mixture as a water/alcohol azeotrop, which can be accomplished either sequentially or substantially simultaneously with step (1); removing the crystal precipitate from the mixture; (4) distilling the impure lactate ester to remove impurities; and (5) recovering the high purity ester.

Acidulation of the fermentation broth prior to esterification results in consumption of reagents and in production of undesired by-product salts.

Liquid-liquid extraction (LLE) proved to be an efficient way to recover carboxylic acids from contaminated aqueous solutions. It is particularly suitable for recovery of acidic fermentation products from fermentation liquors. Thus, a large fraction of the world's citric acid production uses an LLE process to recover the acid from the broth by extraction with an extraction composed of a water immiscible amine in a diluent. This extractant combines high recovery yields, high selectivity and reversibility, resulting in high yields of recovery of pure product at relatively high concentration. As stated hereinbefore, Baniel, et al. (US 4,275,234) have found that the extracted acid can be recovered from the acid comprising extractant (extract) by back-extraction with water. They have also found that, if the back-extraction is conducted at a temperature higher than that of the extraction, the concentration of the acid in the back-extract (the aqueous product of the back-extraction) could be significantly higher than that of the aqueous feed to the process.

Yet, if the concentration of the acid in the feed is very low, the concentration of the back-extract could still be too low. That is particularly true when the feed consists mainly of the salt of the acid rather than the free acid.

Acidulating neutral fermentation liquors by the addition of acids for recovery via ester formation or other methods, results in the formation of by-product salts, such as gypsum in the case of calcium lactate acidulation with sulfuric acid or sodium, or ammonium sulfate in others. Reagents are consumed and disposal of undesired by-products is required. Efforts have recently been made to recover lactic acid from fermentation liquors without formation of by-product salts. Such processes will be referred to hereinbelow as salt splitting processes. In some recently published patents, LLE is applied for salt splitting. Thus, as mentioned above, in U.S. Patent 5,132,456, a strongly basic extractant extracts part of the lactic acid from the neutral solution, which results in a lactic acid loaded extractant and a basic solution. This basic solution, which still comprises most of the lactic acid values, could be recycled as a neutralizing medium to the fermentation. In U.S. 5,510,526 (Baniel, et al.), the extraction of the acid is conducted under CO₂ pressure so that a bicarbonate is formed. The latter can be used as a neutralizing agent in the fermentation. In order to limit the CO₂ pressure to an economic one and still achieve high yields, the extractant used should be quite strong.

In fact, any LLE based salt splitting process that avoids the production of (neutral) by-product salts would require a strongly basic extractant. These extractants are usually composed of an amine as the main active component. The preferred amines are chosen from the group of primary, secondary or tertiary amines, with a total number of at least 18 carbon atoms. Mostly preferred are tertiary amines. A diluent is usually used to achieve the required physical properties. The basicity of the extractant is easily adjusted by adding a polar solvent to the extractant. Such polar solvents enhance the extraction efficiency of the amine, and are usually referred to as enhancers. Alkanols provide very efficient enhancers. The basicity of the extractant is thus adjusted by the amount of the enhancer in the extractant or, more precisely, by the enhancer-to-amine molar ratio. In the strongly basic extractants used in salt splitting processes, the enhancer-to-amine molar ratio is usually at least 1:1, and in many cases is higher than that.

Such strong extractant holds strongly to the extracted lactic acid. Recovery of the extracted acid by washing with an aqueous solution of a base is feasible, but forms the lactate salt of the base. It is therefore not practical in those cases where lactic acid or its esters are the desired product. Back-extraction with water forms a dilute product (back-extract).

As mentioned above, U.S. Patent 5,132,456 suggests a way to recover extracted carboxylic acid from a strong extractant, comprising leaching or back-extraction with an aqueous solution of ammonia or low molecular weight alkyl amine, especially trimethyl amine (TMA). The resultant aqueous ammonium or alkyl ammonium carboxylate solution can be concentrated, if necessary, and the carboxylate can be decomposed thermally to yield the product carboxylic acid and ammonia or amine which can be condensed and recycled. This process is costly and complex, and it is particularly problematic for recovery of extracted lactic acid:
"For lactic acid the decomposition is incomplete, being stopped by the formation of a viscous, almost glassy mass containing polymerized lactic acid along with substantial TMA and water. There are, however, effective ways of driving the decomposition to completion for lactic acid. such as diluting the viscous mass with an appropriate solvent (e.g. methyl isobutyl ketone) and continuing the heating and decomposition process."

With this state of the art in mind, there is now provided, according to the present invention, a process for the recovery of purified lactic acid values from an aqueous feed solution containing lactic acid, lactic acid salt, or mixtures thereof, comprising: a) bringing said feed solution into contact with a substantially immiscible anion exchanger to form a substantially water-immiscible phase comprising an anion exchanger-lactic acid adduct; b) effecting a condensation reaction in said substantially water-immiscible phase between a carboxylic moiety of said lactic acid adduct and a moiety selected from a hydroxyl moiety and a primary or secondary amine moiety to respectively form a lactic acid ester or amide product; and c) separating the formed lactic acid product from the anion exchanger.

In a first preferred aspect of the present invention, there is provided a process for the recovery of purified lactic acid values from an aqueous feed solution containing lactic acid, lactic acid salt, or mixtures thereof, comprising bringing said feed solution into contact with a substantially immiscible anion exchanger to form a substantially water-immiscible phase comprising an anion exchanger-lactic acid adduct, effecting a condensation reaction in said substantially water-immiscible phase between a carboxylic moiety of said lactic acid adduct and a hydroxyl moiety to form a lactic acid ester and separating the formed lactic acid ester from the anion exchanger.

In a second preferred aspect of the present invention there is provided a process for the recovery of purified lactic acid values from an aqueous feed solution containing lactic acid, lactic acid salt, or mixtures thereof, comprising:
a) bringing said feed solution into contact with a substantially immiscible anion exchanger to form a substantially water-immiscible phase comprising an anion exchanger-lactic acid adduct;
b) effecting a condensation reaction in said substantially water-immiscible phase between a carboxylic moiety of said lactic acid adduct and a reagent comprising a primary or secondary amine moiety to form an amide of lactic acid; and c) separating the formed amide of lactic acid from the anion exchanger.

In a first preferred embodiment of the present invention said anion exchanger is a basic extractant comprising a water immiscible amine as the main active component, and there is formed a substantially water-immiscible extract phase comprising an anion exchanger-lactic acid adduct.

In a second preferred embodiment of the present invention said anion exchanger is a solid anion exchanger.

in preferred embodiments of the present invention said aqueous feed solution results from fermentation and is at a pH of at least 4.5.

The process of the present invention is based upon the surprising finding that lactic acid held in a strong extractant can be condensed, thereby losing the acid function, and in preferred embodiments of the present invention the separated lactic acid ester has a purity of at least 99%.

The term "lactic acid values" as used herein is intended to denote lactic acid and its products, mainly condensation products such as esters with alkanols, lactides, oligomers or polymers of lactic acid and amides of lactic acid.

In preferred embodiments of the present invention said lactic acid salt is selected from a group consisting of sodium, calcium, ammonium lactate and mixtures thereof.

In the organic phase formed on extraction, the lactic acid is bound to a basic amine. In fact, it is believed that the organic phase formed on the extraction by an amine comprises, instead of lactic acid, a salt-amine lactate. That is particularly true in the case of an extractant of at least a moderate strength with an apparent basicity corresponding to a pKa of at least 3.5, and in the case where the lactic acid content in the extract is such that the lactic acid-to-amine ratio is lower than one. By analogy, to the above-mentioned DuPont patent, one would expect that a strong acid addition would be required to effect the esterification. The addition of such strong acid would liberate the extracted lactic acid and would allow esterification, but at the cost of forming the amine salt of the strong acid in the organic phase. Regeneration of the amine from that salt would have been required to allow reuse as an extractant. Such regeneration would require contact with a base solution and would form a by-product salt of the strong acid.

Thus, compared to the DuPont process, the process in the present invention has several advantages, including avoidance of by-product salt formation and saving on energy used for water evaporation.

In the condensation reaction, a carboxylic moiety reacts with an hydroxyl moiety to form an ester or with a reagent comprising a primary or secondary amine moiety to form an amide. In both cases a water molecule is formed as a by-product. Thus, the presence of water drives the equilibrium towards the opposite direction, i.e. hydrolysis. In the DuPont process, the fermentation liquor needs to be concentrated prior to the reaction. In the examples used in that patent, the broth is concentrated up to about a water content of 25-28%. This corresponds to distillation of about 6 to 9 tons of water per ton of lactic acid in the product.

In the present process, the condensation is effected in an organic phase which is formed upon bringing the feed solution into contact with an amine based basic extractant. The solubility of water in the extractant is small. Some water co-extracts with the lactic acid, but that is limited to about one mole of water per mole of lactic acid (compared to about 2.5 in the concentrated solution used in the example of the DuPont patent). Thus, the extraction provides for the removal of water in addition to the separation of lactic acid from the impurities, which is equivalent to that obtained by distilling about 7 to 9 tons of water per ton of lactic acid in the product. In addition, water formed in the esterification is removed in the DuPont patent by distilling an azeotrop, while in the present invention the water formed is rejected from the organic phase as it does not dissolve there. Yet, if desired for accelerating the esterification, the system of the present invention lends itself to easy water removal. Co-extracted water can be removed prior to effecting the condensation to leave a practically anhydrous medium. Water formed in the condensation could be removed either sequentially, or substantially simultaneously with the condensation reaction by distillation, as water or as an azeotrop, with possible use of a carrier gas or at sub-atmospheric pressure.

The basicity of water soluble bases is easily determined by their degree of dissociation in an aqueous solution. The basicity of water immiscible extractants is determined indirectly, through their interaction with solutes in an aqueous solution. Thus, the apparent basicity of highly basic extractants can be compared by contacting them with aqueous solutions of NaCI and determining the pH of the aqueous solution in equilibrium. The higher the pH, the stronger the apparent basicity of the extractant. For comparing extractants of medium or weak basicity, equilibration with acid solutions is preferred. Unlike water soluble bases, the apparent basicity found for water immiscible extractants is determined, in addition to the properties of the amine, by the acid in the aqueous solution, by steric hindrance to extraction, and by the diluents of the amine.

In preferred embodiments of the present invention said contact with a substantially immiscible basic extractant forms, in addition to the amine lactic acid adduct, a basic product, which basic product is preferably selected from the group consisting of ammonia, carbonates and bicarbonates of ammonia, of alkali and of alkaline earth metals.

Also preferred are embodiments wherein said contact with a substantially immiscible basic extractant is conducted in a CO₂ atmosphere.

The condensation reaction is accelerated at elevated temperatures. Thus, it is preferably conducted at a temperature of at least 100°C and more preferably at a temperature of at least 140°C.

Said condensation is preferably also conducted in the presence of a catalyst, e.g. tin ions, toluene sulfonic acid, sulfuric acid, titanium, Lewis acid catalysts and non-oxidizing strong acid catalysts.

Similarly, said condensation reaction can be conducted at sub-atmospheric pressure and/or in the presence of a carrier gas.

In the ester forming condensation reaction, the carboxylic moiety of the lactic acid reacts with an hydroxyl moiety. Lactic acid is an hydroxycarboxylic acid. Therefore, the hydroxyl moiety could be on a second lactic acid molecule forming one of the lactic acid condensation products, which could be a dimer, e.g. lactide, trimer or oligomer. The hydroxyl moiety could also be of another molecule, e.g., an alkanol, and in preferred embodiments of the present invention said alkanol is present in the basic extractant used for lactic acid extraction, where it can act as an extraction enhancer, and thereafter said alkanol is present in the extract. In these cases, the alkanol has a double role, as an enhancer and a reagent for esterification. Otherwise, or in addition, an alkanol can be added to the extract prior to effecting the condensation reaction. The added alkanol can be the alkanol used as the enhancer or a different one. Reaction of the extracted lactic acid with an alkanol that also provides an enhancer has an additional benefit: it consumes the alkanol, decreases its proportion in the extract, and thereby releases more lactic acid for further esterification.

In the amide-forming condensation reaction the carboxylic moiety of the lactic acid reacts with a reagent comprising a primary or secondary amine moiety to form an amide. The reagent is preferably of a low molecular weight Such amide should be a product of a reaction with a reagent molecule and not with cation exchanger, which in many cases carry amine functional groups. Therefore these functional groups of the cation exchangers are preferably tertiary or quaternary amines.

USP 5,132,456 uses ammonia or alkyl amine to recover carboxylic acid bound to a sorbing phase. Yet, there the recovery is effected by forming the salt of the carboxylic acid rather than the amide as described in Claim 1: "(c) contacting the separated acid-sorbing phase with an aqueous solution of low molecular weight alkyl amine or ammonia, thereby solubilizing said carboxylic acid from the sorbing phase into said aqueous solution as alkylammonium or ammonium carboxylate,...". The difficulties involved with the formation of these salts were described earlier (see page 9).

On condensation, the lactic acid loses its carboxylic function and thereby detaches from the anion exchanger. The ester or amid formed can be considered a neutral molecule, or at least as having an acidity much lower than the lactic acid. Therefore, it does not interact strongly with the anion exchanger and its separation from the basic extractant is easy. Thus, esterification obviates the need of the very complex and costly recovery process suggested in U.S. Patent 5,132,456.

Water formed in said condensation reaction can be removed either sequentially or substantially simultaneously with said condensation reaction and the lactic acid ester or amide formed in the condensation reaction can be separated from the anion exchanger after effecting the reaction or during the condensation. Separation during the condensation accelerates the reaction. The ester or amide could be separated by transfer into a solvent which is immiscible with the anion exchanger or has low miscibility with it. Alternatively, if the lactate ester or amide has significant solubility in water, it can be washed out of the extract after the condensation to form an aqueous solution of the ester or amide. Another option is distilling the lactic acid ester or amide into the vapor phase, either during the reaction or after it. Such separation into the vapor phase could be assisted by operation at sub-atmospheric pressure or by transfer of a carrier gas. In that case, if an amine containing extractant is used as the anion exchanger, the amine's volatility is preferably lower than that of the ester or amide. Condensation products of suitable molecular weight and polarity may not be soluble in the lactic acid depleted organic phase and may precipitate out of it.

The lactic acid ester could be used as is, e.g. as a "green" solvent; converted to other esters, e.g. through trans-esterification; reacted to form polylactic acid or other polymers or hydrolyzed to recover lactic acid. Thus, said lactic acid ester can be used to form lactoyl lactate or lactide. In preferred embodiments of the present invention said lactide contains less than 20 milliequivalents of free acid and is suitable for direct use in the production of polylactic acid without the need for further purification. In those cases where the ester was formed from lactic acid and an alkanol it is hydrolyzed to lactic acid and the alkanol. Hydrolysis is simply conducted by contact with water or with an aqueous solution. In those cases where the ester is separated into an aqueous solution, hydrolysis could be effected in the ester solution formed and induced by the water presence. The alkanol formed on hydrolysis, as well as on other treatments of the ester such as trans-esterification or condensation, can be separated and reused in a condensation reaction, e.g. by reintroduction into the extractant prior to the extraction of more lactic acid or into the extract.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1.

An extractant composed of 47 wt% tricaprylyl amine (Henkel's Alamine 336), 33 wt% octanol and 20 w% kerosene was contacted with an aqueous solution of lactic acid. An extract containing 11 wt% lactic acid was formed. This extract was heated in a closed pressure vessel to 160°C and kept at this temperature for 4 hours. At the end of the heating, the organic phase contained 15.4 wt% octyl lactate, i.e. more than 60% of the extracted lactic acid reacted with the octanol present in the organic phase to form the ester.

### Example 2.

An extractant composed of 47 wt% tricaprylyl amine (Henkel's Alamine 336), 33 wt% octanol and 20 wt% kerosene was contacted in a pressure vessel with an aqueous solution comprising 50 wt% sodium lactate. The aqueous and organic phases were mixed at ambient temperature under CO₂ pressure of 30 atmospheres for 6 hours. The organic phase was then separated under pressure from the aqueous solution and from the sodium bicarbonate formed in the reaction. It contained 10 wt% lactic acid. Butanol was added to it (38.3g butanol per 78.5g organic phase). The mixture was heated to 166°C in a closed pressure vessel and kept at this tempeature for 6 hours. After cooling the organic phase was analyzed by gas chromatography. About 82% of the lactic acid there was converted to butyl lactate. The latter was practically totally recovered from the organic phase by distillation at 5000 Pa (40 mmHg).

## Claims

1. A process for the recovery of purified lactic acid values from an aqueous feed solution containing lactic acid, lactic acid salt, or mixtures thereof, comprising:
a) bringing said feed solution into contact with a substantially immiscible anion exchanger to form a substantially water-immiscible phase comprising an anion exchanger-lactic acid adduct;
b) effecting a condensation reaction in said substantially water-immiscible phase between a carboxylic moiety of said lactic acid adduct and a moiety selected from a hydroxyl moiety and a primary or secondary amine moiety to respectively form a lactic acid ester or amide product; and
c) separating the formed lactic acid ester or amide product from the anion exchanger.

2. A process according to claim 1, **characterized in that** step b) comprises effecting the condensation reaction in said substantially water-immiscible phase between the carboxylic moiety of said lactic acid adduct and the hydroxyl moiety to form the lactic acid ester; and the formed lactic acid ester is separated from the anion exchanger in step c).

3. A process according to claim 1 or 2, wherein said anion exchanger is a basic extractant comprising a water immiscible amine as the main active component, and there is formed a substantially water-immiscible extract phase comprising an anion exchanger-lactic acid adduct.

4. A process according to any one of the preceding claims, wherein said anion exchanger is a solid anion exchanger.

5. A process according to any one of the preceding claims, wherein said contact with a substantially immiscible anion exchanger is carried out in the presence of an added alkanol.

6. A process according to any one of the preceding claims, wherein steps a), b) and c) are conducted so as to form the separated lactic acid ester with a purity of at least 99%.

7. A process according to any one of the preceding claims, wherein said lactic acid ester is a product of the reaction of at least two lactic acid molecules.

8. A process according to any one of the preceding claims, wherein said lactic acid ester is selected from the group consisting of lactoyl lactate and lactide.

9. A process according to any one of the preceding claims, wherein said lactic acid ester is lactide.

10. A process according to claim 9, wherein said lactide contains less than 20 milliequivalents of free acid.

11. A process according to any one of the preceding claims, wherein said separated lactic acid ester is hydrolyzed to form the lactic acid.

12. A process according to claim 3, wherein said basic extractant comprises an alkanol.

13. A process according to claim 3, wherein alkanol is added to said extract prior to effecting the condensation reaction.

14. A process according to any one of the preceding claims, wherein said lactic acid ester is a product of the reaction of at least one lactic acid molecule and at least one alkanol molecule.

15. A process according to any one of the preceding claims, wherein said separated lactic acid ester is hydrolyzed to form the lactic acid and an alkanol.

16. A process according to claim 15, wherein said alkanol formed upon hydrolyzing said lactic acid ester is reintroduced into said basic extractant prior to extraction of more lactic acid, or into said extract prior to effecting the condensation reaction.

17. A process according to claim 3, wherein co-extracted water is removed from said extract prior to said condensation reaction.

18. A process according to any one of the preceding claims, wherein water formed in said condensation reaction is removed either sequentially or substantially simultaneously with said condensation reaction.

19. A process according to any one of the preceding claims, wherein said lactic acid product is separated from said anion exchanger either sequentially or substantially simultaneously with said condensation reaction.

20. A process according to any one of the preceding claims, wherein said separation is into an aqueous solution.

21. A process according to any one of the preceding claims, wherein said separation is into a solvent, which solvent is immiscible or of low miscibility with the anion exchanger.

22. A process according to any one of the preceding claims, wherein said separation is into the vapor phase.

23. A process according to claim 20, wherein separation into said aqueous solution induces hydrolysis.

24. A process according to any one of the preceding claims, wherein said condensation reaction is conducted at a temperature higher than 100°C.

25. A process according to any one of the preceding claims, wherein said condensation reaction is conducted at sub-atmospheric pressure.

26. A process according to any one of the preceding claims, wherein said condensation reaction is conducted in the presence of a carrier gas.

27. A process according to any one of the preceding claims, wherein said anion exchanger is at least a moderately strong base, with an apparent basicity corresponding to a pKa of at least 3.5.

28. A process according to any one of the preceding claims, wherein said anion exchanger is at least a moderately strong base, with an apparent basicity corresponding to a pKa of at least 4.

29. A process according to any one of the preceding claims, wherein the lactic acid-to-amine molar ratio in said extract is less than one.

30. A process according to any one of the preceding claims, wherein steps a), b) and c) are conducted such that the purity of the recovered lactic acid values is higher than 99%.

31. A process according to any one of the preceding claims, wherein said lactic acid ester has low solubility in the extract.

32. A process according to any one of the preceding claims, wherein said lactic acid ester is used in reactions selected from the group comprising trans-esterification and condensations resulting in polylactate.

33. A process according to any one of the preceding claims, wherein said aqueous feed solution results from fermentation.

34. A process according to any one of the preceding claims, wherein said lactic acid salt is selected from a group consisting of sodium, calcium, ammonium lactate and mixtures thereof.

35. A process according to any one of the preceding claims, wherein said contact with a substantially immiscible anion exchanger forms, in addition to the lactic acid adduct, a basic product.

36. A process according to claim 35, wherein said basic product is selected from the group consisting of ammonia, carbonates and bicarbonates of ammonia, of alkali and of alkaline earth metals.

37. A process according to any one of the preceding claims, wherein said contact with a substantially immiscible anion-exchanger is conducted in a CO₂ atmosphere.

38. A process according to any one of the preceding claims, wherein said aqueous feed solution is at pH of at least 4.5.

39. A process according to any one of the preceding claims, wherein said condensation is conducted in the presence of a catalyst.

40. A process according to claim 1, **characterized in that** step b) comprises effecting the condensation reaction in said substantially water-immiscible phase between the carboxylic moiety of said lactic acid adduct and a reagent comprising the primary or secondary amine moiety to form an amide of lactic acid; and the formed amide of lactic acid is separated from the anion exchanger in step c).

## Patentansprüche

1. Verfahren zur Gewinnung von gereinigten Milchsäure-Wertstoffen aus einer wässrigen Beladungslösung, enthaltend Milchsäure, Milchsäure-Salz oder Mischungen davon, welches Verfahren umfasst:
a) Kontaktieren der Beladungslösung mit einem im Wesentlichen nicht mischbaren Anionenaustauscher, um eine mit Wasser im Wesentlichen nicht mischbare Phase zu erzeugen, die ein Anionenaustauscher-Milchsäure-Addukt aufweist;
b) Herbeiführen einer Kondensationsreaktion in der mit Wasser im Wesentlichen nicht mischbaren Phase zwischen einem Carboxyl-Teil des Milchsäure-Adduktes und einem Teil, ausgewählt aus der Gruppe, bestehend aus einem Hydroxyl-Teil und einem primären oder sekundären Amin-Teil, zu der jeweiligen Form eines Milchsäureester- oder Amid-Produktes; sowie
c) Abtrennen des erzeugten Milchsäureester- oder Amid-Produktes von dem Anionenaustauscher.

2. Verfahren nach Anspruch 1, **dadurch gekennzeichnet, dass** Schritt b) das Herbeiführen der Kondensationsreaktion in der mit Wasser weitgehend nicht mischbaren Phase zwischen dem Carboxyl-Teil des Milchsäure-Addukts und dem Hydroxyl-Teil umfasst, um den Milchsäureester zu erzeugen; und dass in Schritt c) der erzeugte Milchsäureester von dem Anionenaustauscher abgetrennt wird.

3. Verfahren nach Anspruch 1 oder 2, bei welchem der Anionenaustauscher ein basisches Extraktionsmittel ist, aufweisend ein mit Wasser nicht mischbares Amin als die aktive Hauptkomponente und bei welchem eine mit Wasser im Wesentlichen nicht mischbare Extraktphase gebildet wird, die ein Anionenaustauscher-Milchsäure-Addukt aufweist.

4. Verfahren nach einem der vorgenannten Ansprüche, bei welchem der Anionenaustauscher ein fester Anionenaustauscher ist.

5. Verfahren nach einem der vorgenannten Ansprüche, bei welchem der Kontakt mit einem im Wesentlichen nicht mischbaren Anionenaustauscher in Gegenwart eines zugesetzten Alkanols vorgenommen wird.

6. Verfahren nach einem der vorgenannten Ansprüche, bei welchem die Schritte a), b) und c) so ausgeführt werden, dass der abgetrennte Milchsäureester mit einer Reinheit von mindestens 99% erzeugt wird.

7. Verfahren nach einem der vorgenannten Ansprüche, bei welchem der Milchsäureester ein Produkt der Reaktion von mindestens zwei Milchsäure-Molekülen ist.

8. Verfahren nach einem der vorgenannten Ansprüche, bei welchem der Milchsäureester ausgewählt wird aus der Gruppe, bestehend aus Lactyllactat und Lactid.

9. Verfahren nach einem der vorgenannten Ansprüche, bei welchem der Milchsäureester Lactid ist.

10. Verfahren nach Anspruch 9, bei welchem das Lactid weniger als 20 mval freie Säure enthält.

11. Verfahren nach einem der vorgenannten Ansprüche, bei welchem der abgetrennte Milchsäureester hydrolysiert wird, um die Milchsäure zu erzeugen.

12. Verfahren nach Anspruch 3, bei welchem das basische Extraktionsmittel ein Alkanol aufweist.

13. Verfahren nach Anspruch 3, bei welchem Alkanol dem Extrakt zugesetzt wird, bevor die Kondensationsreaktion ausgeführt wird.

14. Verfahren nach einem der vorgenannten Ansprüche, bei welchem der Milchsäureester ein Produkt der Reaktion mindestens eines Milchsäure-Moleküls und mindestens eines Alkanol-Moleküls ist.

15. Verfahren nach einem der vorgenannten Ansprüche, bei welchem der abgetrennte Milchsäureester hydrolysiert wird, um die Milchsäure und einem Alkanol zu erzeugen.

16. Verfahren nach Anspruch 15, bei welchem das Alkanol, das beim Hydrolysieren des Milchsäureesters gebildet wird, wieder in das basische Extraktionsmittel vor der Extraktion weiterer Milchsäure eingeführt wird oder in den Extrakt eingeführt wird, bevor die Kondensationsreaktion ausgeführt wird.

17. Verfahren nach Anspruch 3, bei welchem das coextrahierte Wasser aus dem Extrakt vor der Kondensationsreaktion entfernt wird.

18. Verfahren nach einem der vorgenannten Ansprüche, bei welchem das Wasser, das in der Kondensationsreaktion erzeugt wird, entweder sequentiell oder im Wesentlichen gleichzeitig mit der Kondensationsreaktion entfernt wird.

19. Verfahren nach einem der vorgenannten Ansprüche, bei welchem das Milchsäure-Produkt von dem Anionenaustauscher entweder sequentiell oder im Wesentlichen gleichzeitig mit der Kondensationsreaktion abgetrennt wird.

20. Verfahren nach einem der vorgenannten Ansprüche, bei welchem die Abtrennung in eine wässrige Lösung hinein erfolgt.

21. Verfahren nach einem der vorgenannten Ansprüche, bei welchem die Abtrennung in ein Lösemittel hinein erfolgt, wobei das Lösemittel mit dem Anionenaustauscher unmischbar ist oder eine geringe Mischbarkeit hat.

22. Verfahren nach einem der vorgenannten Ansprüche, bei welchem die Abtrennung in die Dampfphase hinein erfolgt.

23. Verfahren nach Anspruch 20, bei welchem die Abtrennung in die wässrige Lösung hinein eine Hydrolyse einleitet.

24. Verfahren nach einem der vorgenannten Ansprüche, bei welchem die Kondensationsreaktion bei einer Temperatur oberhalb von 100°C ausgeführt wird.

25. Verfahren nach einem der vorgenannten Ansprüche, bei welchem die Kondensationsreaktion bei Unterdruck ausgeführt wird.

26. Verfahren nach einem der vorgenannten Ansprüche, bei welchem die Kondensationsreaktion in Gegenwart eines Trägergases ausgeführt wird.

27. Verfahren nach einem der vorgenannten Ansprüche, bei welchem der Anionenaustauscher mindestens eine mäßig starke Base mit einer scheinbaren Basizität entsprechend einem pKa-Wert von mindestens 3,5 ist.

28. Verfahren nach einem der vorgenannten Ansprüche, bei welchem der Anionenaustauscher mindestens eine mäßig starke Base mit einer scheinbaren Basizität entsprechend einem pKa-Wert von mindestens 4 ist.

29. Verfahren nach einem der vorgenannten Ansprüche, bei welchem das Molverhältnis Milchsäure/Amin in dem Extrakt kleiner ist als 1.

30. Verfahren nach einem der vorgenannten Ansprüche, bei welchem die Schritte a), b) und c) so ausgeführt werden, dass die Reinheit der gewonnenen Milchsäure-Wertstoffe größer ist als 99%.

31. Verfahren nach einem der vorgenannten Ansprüche, bei welchem der Milchsäureester in dem Extrakt eine geringe Löslichkeit hat.

32. Verfahren nach einem der vorgenannten Ansprüche, bei welchem der Milchsäureester in Reaktionen verwendet wird, ausgewählt aus der Gruppe, umfassend Umesterung und Kondensationen, die zu Polylactat führen.

33. Verfahren nach einem der vorgenannten Ansprüche, bei welchem die wässrige Beladungslösung aus der Fermentation resultiert.

34. Verfahren nach einem der vorgenannten Ansprüche, bei welchem das Milchsäure-Salz ausgewählt wird aus der Gruppe, bestehend aus Natrium-, Calcium-, Ammoniumlactat und Mischungen davon.

35. Verfahren nach einem der vorgenannten Ansprüche, bei welchem der Kontakt mit einem im Wesentlichen nicht mischbaren Anionenaustauscher zusätzlich zu dem Milchsäure-Addukt ein basisches Produkt erzeugt.

36. Verfahren nach Anspruch 35, bei welchem das basische Produkt ausgewählt wird aus der Gruppe, bestehend aus Ammoniak, Carbonaten und Hydrogencarbonaten von Ammoniak der Alkali- und Erdalkalimetalle.

37. Verfahren nach einem der vorgenannten Ansprüche, bei welchem der Kontakt mit einem im Wesentlichen nicht mischbaren Anionenaustauscher in einer CO₂-Atmosphäre ausgeführt wird.

38. Verfahren nach einem der vorgenannten Ansprüche, bei welchem die wässrige Beladungslösung einen pH-Wert von mindestens 4,5 hat.

39. Verfahren nach einem der vorgenannten Ansprüche, bei welchem die Kondensation in Gegenwart eines Katalysators ausgeführt wird.

40. Verfahren nach Anspruch 1, **dadurch gekennzeichnet, dass** Schritt b) das Herbeiführen der Kondensationsreaktion in der im Wasser weitgehend nicht mischbaren Phase zwischen dem Carboxyl-Teil des Milchsäure-Adduktes und einem Reagenz umfasst, das den primären oder sekundären Amin-Teil aufweist, um ein Amid der Milchsäure zu erzeugen; und in Schritt c) das erzeugte Amid der Milchsäure von dem Anionenaustauscher abgetrennt wird.

## Revendications

1. Procédé pour récupérer de l'acide lactique purifié à partir d'une solution aqueuse d'alimentation contenant de l'acide lactique, un sel d'acide lactique ou des mélanges de ceux-ci, comprenant :
a) la mise en contact de ladite solution d'alimentation avec un échangeur d'anions sensiblement immiscible pour former une phase sensiblement immiscible à l'eau comprenant un produit d'addition échangeur d'anions-acide lactique ;
b) la conduite d'une réaction de condensation dans ladite phase sensiblement immiscible à l'eau entre une entité carboxylique dudit produit d'addition d'acide lactique et une entité choisie parmi une entité hydroxyle et une entité amine primaire ou secondaire pour former respectivement un produit constitué par un ester ou un amide de l'acide lactique ; et
c) la séparation du produit constitué par un ester ou amide de l'acide lactique formé d'avec l'échangeur d'anions.

2. Procédé selon la revendication 1, **caractérisé en ce que** l'étape b) comprend la conduite de la réaction de condensation dans ladite phase sensiblement immiscible à l'eau entre l'entité carboxylique dudit produit d'addition d'acide lactique et l'entité hydroxyle pour former l'ester d'acide lactique ; et l'ester d'acide lactique formé est séparé de l'échangeur d'anions dans l'étape c).

3. Procédé selon la revendication 1 ou 2, où ledit échangeur d'anions et un agent d'extraction basique comprenant une amine immiscible à l'eau comme composant actif principal, et il se forme une phase d'extrait sensiblement immiscible à l'eau comprenant un produit d'addition échangeur d'anions-acide lactique.

4. Procédé selon l'une quelconque des revendications précédentes, où ledit échangeur d'anions est un échangeur d'anions solide.

5. Procédé selon l'une quelconque des revendications précédentes, où ledit contact avec un échangeur d'anions sensiblement immiscible est réalisé en présence d'un alcanol ajouté.

6. Procédé selon l'une quelconque des revendications précédentes, où les étapes a), b) et c) sont mises en oeuvre de manière à former l'ester d'acide lactique séparé avec une pureté d'au moins 99 %.

7. Procédé selon l'une quelconque des revendications précédentes, où ledit ester d'acide lactique est un produit de la réaction d'au moins deux molécules d'acide lactique.

8. Procédé selon l'une quelconque des revendications précédentes, où ledit ester d'acide lactique est choisi dans le groupe consistant en le lactate de lactoyle et le lactide.

9. Procédé selon l'une quelconque des revendications précédentes, où ledit ester d'acide lactique est le lactide.

10. Procédé selon la revendication 9, où ledit lactide contient moins de 20 milliéquivalents d'acide libre.

11. Procédé selon l'une quelconque des revendications précédentes, où ledit ester d'acide lactique séparé est hydrolysé pour former l'acide lactique.

12. Procédé selon la revendication 3, où ledit agent d'extraction basique comprend un alcanol.

13. Procédé selon la revendication 3, où un alcanol est ajouté audit extrait avant la conduite de la réaction de condensation.

14. Procédé selon l'une quelconque des revendications précédentes, où ledit ester d'acide lactique est un produit de la réaction d'au moins une molécule d'acide lactique et d'au moins une molécule d'alcanol.

15. Procédé selon l'une quelconque des revendications précédentes, où ledit ester d'acide lactique séparé est hydrolysé pour former l'acide lactique et un alcanol.

16. Procédé selon la revendication 15, où ledit alcanol formé par hydrolyse dudit ester d'acide lactique est réintroduit dans ledit agent d'extraction basique avant l'extraction d'un supplément d'acide lactique, ou dans ledit extrait avant la conduite de la réaction de condensation.

17. Procédé selon la revendication 3, où l'eau co-extraite est retirée dudit extrait avant ladite réaction de condensation.

18. Procédé selon l'une quelconque des revendications précédentes, où l'eau formée dans ladite réaction de condensation est retirée successivement ou sensiblement simultanément avec ladite réaction de condensation.

19. Procédé selon l'une quelconque des revendications précédentes, où ledit produit constitué par l'acide lactique est séparé dudit échangeur d'anions successivement ou sensiblement simultanément avec ladite réaction de condensation.

20. Procédé selon l'une quelconque des revendications précédentes, où ladite séparation est dans une solution aqueuse.

21. Procédé selon l'une quelconque des revendications précédentes, où ladite séparation est dans un solvant, lequel solvant est immiscible ou faiblement miscible à l'échangeur d'anions.

22. Procédé selon l'une quelconque des revendications précédentes, où ladite séparation est dans la phase gazeuse.

23. Procédé selon la revendication 20, où la séparation dans ladite solution aqueuse induit une hydrolyse.

24. Procédé selon l'une quelconque des revendications précédentes, où ladite réaction de condensation est conduite à une température supérieure à 100°C.

25. Procédé selon l'une quelconque des revendications précédentes, où ladite réaction de condensation est conduite à une pression inférieure à la pression atmosphérique.

26. Procédé selon l'une quelconque des revendications précédentes, où ladite réaction de condensation est conduite en présente d'un gaz vecteur.

27. Procédé selon l'une quelconque des revendications précédentes, où ledit échangeur d'anions est au moins une base modérément forte ayant une basicité apparente correspondant à un pKa d'au moins 3,5.

28. Procédé selon l'une quelconque des revendications précédentes, où ledit échangeur d'anions est au moins une base modérément forte ayant une basicité apparente correspondant à un pKa d'au moins 4.

29. Procédé selon l'une quelconque des revendications précédentes, où le rapport molaire acide lactique à amine dans ledit extrait est inférieur à 1.

30. Procédé selon l'une quelconque des revendications précédentes, où les étapes a), b) et c) sont mises en oeuvre de manière que la pureté de l'acide lactique récupéré soit supérieure à 99 %.

31. Procédé selon l'une quelconque des revendications précédentes, où ledit ester d'acide lactique a une faible solubilité dans l'extrait.

32. Procédé selon l'une quelconque des revendications précédentes, où ledit ester d'acide lactique est utilisé dans des réactions choisies dans le groupe comprenant les transestérifications et les condensations conduisant à du polylactate.

33. Procédé selon l'une quelconque des revendications précédentes, où ladite solution aqueuse d'alimentation résulte d'une fermentation.

34. Procédé selon l'une quelconque des revendications précédentes, où ledit sel d'acide lactique est choisi dans un groupe consistant en le lactate de sodium, de calcium, d'ammonium et leurs mélanges.

35. Procédé selon l'une quelconque des revendications précédentes, où ledit contact avec un échangeur d'anions sensiblement immiscible forme un produit basique en plus du produit d'addition d'acide lactique.

36. Procédé selon la revendication 35, où ledit produit basique est choisi dans le groupe consistant en l'ammoniac, les carbonates et bicarbonates d'ammoniac, de métaux alcalins et alcalino-terreux.

37. Procédé selon l'une quelconque des revendications précédentes, où ledit contact avec un échangeur d'anions sensiblement immiscible est réalisé dans une atmosphère de CO₂.

38. Procédé selon l'une quelconque des revendications précédentes, où ladite solution aqueuse d'alimentation est à un pH d'au moins 4,5.

39. Procédé selon l'une quelconque des revendications précédentes, où ladite condensation est conduite en présence d'un catalyseur.

40. Procédé selon la revendication 1, **caractérisé en ce que** l'étape b) comprend la conduite de la réaction de condensation dans ladite phase sensiblement immiscible à l'eau entre l'entité carboxylique dudit produit d'addition d'acide lactique et un réactif comprenant l'entité amine primaire ou secondaire pour former un amide d'acide lactique ; et l'amide d'acide lactique formé est séparé de l'échangeur d'anions dans l'étape c).
